Europäisches Patentamt

(19) European Patent Office

Office européen des brevets

(11) Publication number: **0 074 160**
**B1**

(12) **EUROPEAN PATENT SPECIFICATION**

(45) Date of publication of patent specification: **01.02.89**

(21) Application number: **82302800.6**

(22) Date of filing: **01.06.82**

(51) Int. Cl.⁴: **A 01 G 7/04, A 61 N 1/32**

(54) A physical treatment for influencing biological activity.

(30) Priority: **02.06.81 GB 8116828**

(43) Date of publication of application:
**16.03.83 Bulletin 83/11**

(45) Publication of the grant of the patent:
**01.02.89 Bulletin 89/05**

(84) Designated Contracting States:
**AT BE CH DE FR IT LI LU NL SE**

(56) References cited:
**DE-A-2 812 546**
**DE-A-2 841 933**
**FR-A-2 433 901**
**US-A-3 893 462**
**US-A-4 055 915**
**US-A-4 105 017**

(73) Proprietor: **MEHESZ CORPORATION**
**Vaduz (LI)**

(72) Inventor: **Mehesz, Stephen Von**
**Hope Lodge 10 Great Western Road**
**Dorchester Dorset (GB)**

(74) Representative: **Jenkins, Richard Gray et al**
**R.G.C. Jenkins & Co. 26 Caxton Street**
**London SW1H 0RJ (GB)**

## Description

This invention relates to apparatus for influencing biological activity.

The present invention relates to apparatus for influencing biological activity by generating electrically dependent oscillations via a liquid medium with which living organisms are in contact so as to influence their physiology. Both plant and animal tissues may be subjected to treatment using the apparatus.

US-A-4105017 discloses an apparatus for altering the behaviour of living tissues, primarily promoting bone growth and repair. For this purpose, it utilizes signals which have positive and negative going portions which are required to be substantially different from each other both in amplitude and in length. These oscillatory signals are applied to the tissue being treated by means of an inductive coil placed adjacent to the tissue. The coil generates a magnetic field which penetrates the tissue and induces current within the tissue. The specification emphasises the disadvantages of using implanted electrodes (column 2, line 45), electrodes on (i.e. presumably simply in contact with) tissues or cells (column 9, lines 2 and 3), capacitatively coupled signals (column 2, line 53) and electrostatic fields (column 9, line 1). It emphasises that it is the specific types of unsymmetrical waveforms, applied specifically by inductive coils to the tissues, that achieve the particular benefits suggested in that prior specification.

According to the present invention we provide apparatus for influencing biological activity comprising:

means for generating an oscillating voltage;

control means for enabling intermittent generation of the oscillating voltage in the form of pulses comprising series of oscillations, the control means being operable so that the duration of the pulses and the duration of the intervals between pulses is independently selectable,

characterised in that;

two movable electrodes are connected to the output of the generating means to develop from the oscillating voltage an oscillating electrical field, extending from one electrode to the other, for application to a biological subject either through a liquid medium contacting the biological subject or by direct contact with the biological subject;

the generating means is adapted to produce the oscillating voltage at a frequency of 40 -7,500 Hz;

and the electrical field oscillations are simple square waves.

Preferably, the duration of said pulses and of said intervals between said pulses is in the range of 0.001-10 seconds.

The output voltage between said electrodes may be in the range 2-30 volts and the apparatus may comprise means for varying said output voltage. The frequency of the oscillations may also be varied.

Certain terms used for convenience hereinafter will now be described, the word "Mehesz" in these terms referring to the inventor of this invention. The "Mehesz Apparatus" is the apparatus in accordance with the invention as defined above. It may comprise two or more electrodes which may either be in a treatment tank or "conducting pads" as defined below. The electric field between the electrodes may thus be applied to the subject either through an electrically conductant liquid medium contained in the tank, or such a medium absorbed in the pads. The liquid medium which may be tap water serves as a coupling agent between the oscillations and the tissues. The "conducting pad" may be a flexible metallic plate jacketed with fibrous material which absorbs water that in turn conducts electricity.

The "Mehesz Effect", is an effect detectable by simple bio-assay, e.g by holding the human hand in the medium in the tank and experiencing a tingling sensation via the nervous system when the oscillations are generated in the medium.

The "Mehesz Treatment", consists of subjecting any biological tissue to the Mehesz Effect. Controlled experiments can be used to demonstrate that certain tissues respond to exposure to the Mehesz Effect by demonstrating differences in their growth pattern compared with comparable tissues placed in the medium in the apparatus for a comparable time but without the Mehesz Effect being administered. The changes in growth pattern may be positive or negative, and whilst accelerated or enhanced growth is the usual aim of the experiment (e.g to generate additional yield or to get the same yield in a shorter time) there are circumstances where a slowing down of growth is desirable (e.g amenity grass to save mowing).

Biological activity is controlled by flux of electrochemical energy within the cell so it is surmised that the Mehesz Treatment modifies metabolic (both anabolic and catabolic) processes. Observations have thus far only been made on multicellular organisms, and it has not yet been determined whether cells increase their individual size in addition to the assumed increased rate of cell division when growth is accelerated. Measurements of cell size and work with unicellular organisms and cell components will elucidate this point.

The apparatus may also comprise a signal amplifier between the generating means and the electrodes for amplifying the signal to the electrodes, with the control means disposed between the generating means and the amplifier.

The apparatus may also conveniently include monitoring equipment to enable various parameters to be measured, for example means for monitoring the frequency and amplitude of the signal and the voltage and current passing between the electrodes. The electronic units may all be solid state components with crystal generated digital output where meansurements are necessary.

The invention will now be further described by way of example with reference to the accompanying drawings in which:-

Fig. 1 illustrates an apparatus for supplying an amplified, pulsed, audio frequency signal to electrodes immersed in a tank,

Fig. 2 illustrates a modification of part of the apparatus shown in Fig. 1 and

Fig. 3 illustrates a further modification of part of the apparatus shown in Fig. 1.

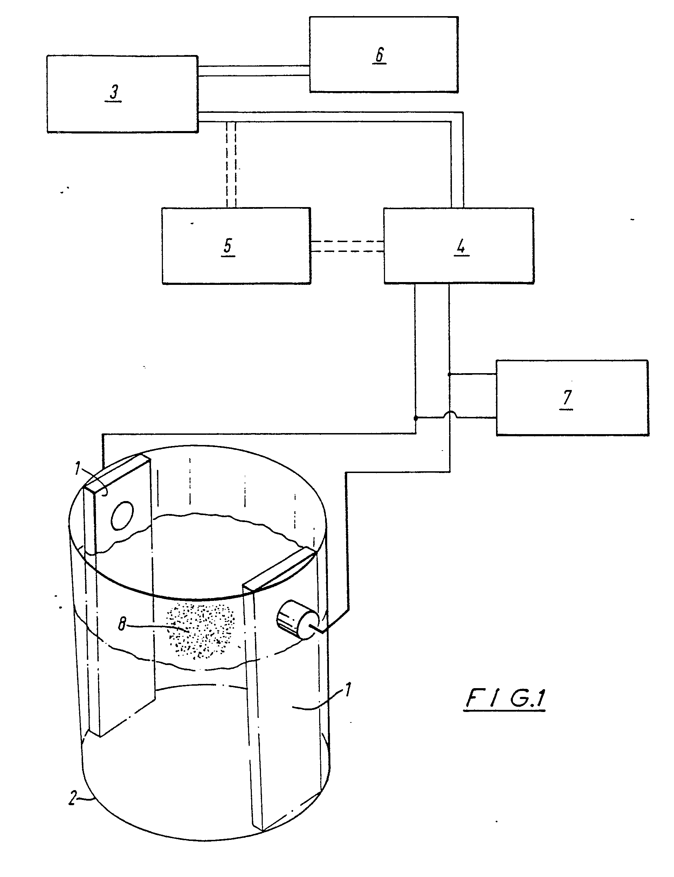

Referring first to Fig. 1, a cylindrical tank 2 is of transparent acrylic plastic which is self-insulating. Two electrodes 1 are fitted vertically in the tank at 180° to each other. They are made of metal of high electrical conductivity, and are bolted through the drilled tank and secured by external brass electrodes. The electrodes need not necessarily be of equal dimension or shape. For example one electrode could be a hollow cylinder fitted inside the tank with the second a much slimmer solid cylinder vertically positioned in the centre. A screw-cap lid (not shown) may be incorporated for use when an integral or magnetically induced stirrer is incorporated. The base may be fitted with a heating element (not shown) incorporating a sensitive (+/-0-5°C) thermostat for accurate control of the temperature of the liquid. All the electronic units are solid state, with crystal-generated digital output where measurements are necessary. The electrodes may be of any appropriate conducting material, being selected with care as potential contamination or heavy metal sensitivity must be borne in mind. Traces of metal from oxidative and other processes affecting the electrodes in the conducting medium could affect very sensitive tissues such as animal cell cultures, eggs, protoplasts or even much larger and tougher tissues such as seeds when a deficiency of an element may be affecting performance. Copper and zinc for example, are well known fungicides, and their use could affect disease scores. Aluminium has been used successfully for electrodes as once a protective layer of aluminium oxide has formed the metal is virtually inert.

A signal generator 3 as a square wave output. The amplitude is variable and both this and the wave form may be observed directly where an oscilloscope is incorporated. A continuous control is used to change the wavelength, and this is shown on a digital, liquid crystal display 6. The generator is self-compensating for fluctuations in mains outputs voltage. The frequency of the wave generated can be varied. Under normal operation conditions it seems that for an appreciable response detectable by the human hand the responses most effective range from approximately 40 to 7,500 Hz. It is advisable to utilise a separate signal amplifier 4 of robut construction and adequate power rather than use the relatively delicate signal generator to drive the signal, especially when large volume tanks are used. A 16 watt amplifier, for example, is quite adequate for treating a 25 litre tank filled with sugarbeet seed. Thus the signal generator 3 is electrically connected to the electrodes 1 via the signal amplifier 4. The apparatus may also be fitted with a lead to a loudspeaker (not shown) for audible assessment of the true audio frequency. Additionally a synthetic sound generator of fixed frequency may be fitted to give an audio signal via the loudspeaker and allow monitoring of pulsed output.

Some tissues appear to respond better to a pulsed signal rather than a continuous one. A pulse generator 5 has separate dual (paired) controls for the "on" and "off" states so that the Mehesz Effect can be brought into activity discontinuously in accurately controlled repeated fashion. The prototype pulse control is capable of random or regular intervals, with the "on" pulses having a duration of from 0.001 second to 10 seconds, and the "off" or "quiet" periods are similarly variable via a series of click-stop switches. Thus the "on" and "off" periods may be equal or unequal in length, and great numbers of exposure combinations are available to the operator. The pulse generator 5 is connected between the signal generator and the amplifier. A voltmeter 7 measures the potential difference between the electrodes and an ammeter is incorporated as a safety device in the event of conductivity being changed as a result of electrolytes leaching into the medium.

The following comments relates to operating the apparatus.

Some information is needed for pre-setting certain paraments, e.g. temperature, wavelength or time. For poultry eggs, for example, the fertile eggs must be kept at incubator temperature throughout the treatment to avoid cold shock, and thermostatic control of the heating element is essential. With dormant material, e.g. plant seeds, ambient temperature is generally adequate, although there may be an advantage in using hot water at non-lethal temperatures to combine the treatment with fungicidal activity or to dissolve chemical inhibitors of germination. There may be benefits in pre-soaking the seeds to initiate breaking of dormancy and commencement of mobilising food reserves by enzymic action. Routine treatment of sugarbeet seeds, for example, has been done at 18°C, in tap water, at 500 Hz and given a positive response in the case of some cultivars and seed stocks with exposures between 30 and 60 minutes continuous treatment and with an output voltage of c. 10V. showing across the terminals. Such a setting induces a very considerable tingle — the Mehesz Effect — if the hand is immersed, and the field appears maximal on the axis between the electrodes. Hence, a stirrer is needed to ensure equal exposure when the tank has more than a small amount of tissue. Single eggs or small quantities of seed 8 may be suspended between the eletrodes in a perforated container (not shown) to ensure repeatable treatment. All settings should be fixed and the circuits allowed to stabilize before immersion of the tissues. The presence of the Mehesz Effect may easily be checked by inserting an extended hand, which should be scrupulously clean, into the tank and altering the plane. An effect governed by c. 10 V. is as much as can be tolerated by the average person.

The nature of the Mehesz Effect will now be described.

The Mehesz Effect is produced by using an apparatus pass a signal of audio frequency between the electrodes. It was first termed "insonation". The physical effect was much greater than that expected from a simple exposure to a low voltage electrical oscillation. The tingling sensation is independent of the current flowing, this being related to the impedance of the fluid. If the resistance of, say, tap water, is lowered by adding salts the measurable current may be increased x 1,000 without affecting the sensation.

The effect does, however, appear to depend upon the wavelength and amplitude of the signal, and at given Hz (frequency) and amplitude values the "Mehesz Dosage" is defined as the product of voltage and time e.g. 10 volts for 30 minutes is equivalent to 300 "Me units". Within certain limits the product curve (V x time) appears to be linear i.e. 10 V x 30 minutes equals 15 V x 20 minutes. Obviously other effects must be taken into account: the robustness of the circuits will determine maximum values of potential difference and amperage, and the physiological state of the tissue is important. Dry, hard, seeds for example have a different sensitivity to soaked seeds in which the germination process has been initiated and in which cell division may be actively occurring. 1,000 V for 0.3 minutes will not produce instantly germinated seeds, but 1 V for 5 hours may germinate, say, ripe cress seed if other conditions such as temperature are optimal because we know that water alone over such a long period may be effective.

Nevertheless the short, powerful treatment may influence subsequent growth and ultimate performance: with dormant seed it might affect the charge on essential molecules such as those in cell membranes. It could conceivably kill actively growing seeds.

In experiments so far the voltage range used has varied from 2 V to 30 V and may be C.17.5 V.

The human hand has been used as a detector and water as the coupling agent because all living cells contain water and protoplasm has an aqueous base. Even very dry cereal seed rarely has less than C.8% water and when harvested under good conditions contains 12 to 18%. Water molecules are vital in the photosynthetic and respiratory processes, and the modest electrical effects of the Mehesz Treatment can be envisaged as influencing the electron transfer processes of energy capture and release.

It is also presumed that oxygen is necessary for activity, and the relative importance of water and oxygen may be ascertained using a non-aqueous fluorocarbon liquids in place of water.

Should an aqueous medium be absolutely necessary it may be possible to utilise a chamber in which a fine spray or mist is used as the conductor yet the biological material be kept relatively dry. A high voltage/low exposure combination would help in this situation - useful in saving time and energy in returning seeds to a proper moisture content for storage.

It is important to know how long any benefit of the Mehesz treatment lasts when applied to organisms in the resting phase e.g. to seeds which are not undergoing cell division and in which mobilisation of stored food is proceeding just fast enough to allow basic respiration and maintain life.

If the Mehesz treatment is comparable with recharging an electrical battery then the mitochondria and various cell membranes are the obvious units to examine for comparison with untreated material. The former, minute (c. 10 nm x 1 nm) organelles are the power houses of the cell and contain their own hereditary material. They can be extracted from ground up tissue by ultracentrifugation and their ability to consume oxygen and substrates measured in vitro. The wholeness or otherwise of the phospholipid membranes of the cell also reflects the strength of the charges and bonds on and between the molecules, and can be checked by electron microscopy.

If the Mehesz Effect does indeed function in this way it is quite conceivable that treated seed will last better in storage and show more vigour upon germination — and perhaps throughout the life cycle — than untreated seed. On the other hand any terminal advantage found may result from a single, initial "push start" advantage giving the equivalent of a slightly longer growth period.

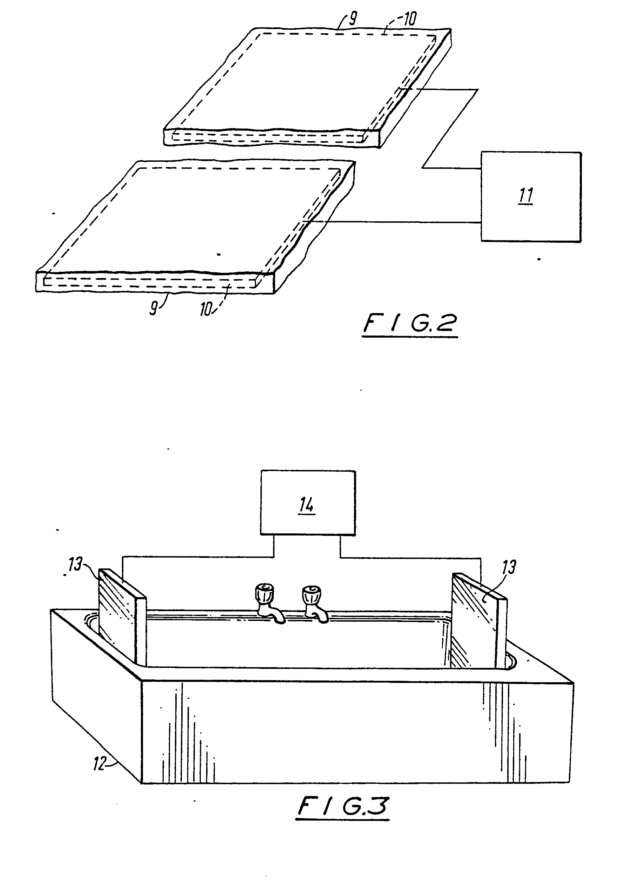

While an important use of the apparatus of Fig. 1 is the treatment of seeds, the apparatus of Fig. 2 may be used for the treatment of humans or animals and that of Fig. 3 for the treatment of humans.

Fig. 2 shows a pair of flexible pads 9, 9 made of a material capable of absorbing an electrically conducting liquid medium, such as tap water. Enclosed within each pad 9 is a thin flexible metal plate 10, which may be made of copper or aluminium and which serves as an electrode. The electrodes 10 are electrically connected to a source of audio frequency electric signals 11 representing the units. In 3 to 5 and 7 shown in Fig. 1 the flexible pads may be moistened with tap water and applied to humans or animals requiring treatment, for example by being wrapped around limbs thereof, and the electric signals applied to the electrodes. The water in the pads acts as a coupling agent so that the Mehesz treatment may be effected on the humans or animals.

The treatment may be medical or non-medical. It has been found that the treatment is beneficial to arthritis sufferers, who have experienced a reduction in pain and/or an increase in mobility after treatment. The treatment is also beneficial in improving the fitness of healthy people, for example by toning up their muscles. Amongst animals, race horses have been successfully treated to improve their mobility.

Fig. 3 shows a domestic bath 12 having electrodes 13 immersed in the bath at opposite ends. The electrodes 13 are electrically connected to a source of audio frequency electric signals 14 representing the units 3 to 5 shown. in Fig. 1. The electrodes 13, connected to the signal source 14 may be fitted to an existing bath as and when required or the electrodes 13 may be left in position in the bath and the signal source connected as required. In using the apparatus, the person to be treated is immersed in water in the bath between the electrodes and the signals applied progressively to the electrodes. The treatment is particularly intended for increasing the fitness of fit persons and physiotherapy.

In another application of the invention instead of immersing the tissue in water or another coupling agent in the tank while the Mehesz Treatment is applied, the apparatus could be operated with water (or an alternative medium) alone in the tank. The resultant (i.e. treated) water might then be brought into contact with living tissues. For example seeds could be germinated in it or have the treated water sprayed on, or growing plants could be watered with treated water to modify growth. Similarly eggs may be brought into contact with the treated water prior to hatching, or the treated water may be administered to animals or fish to modify their growth.

If the electrical signal applied to the electrodes of the apparatus of the invention is not a simple signal, for example where the signal includes a succession of different frequencies or is indeed a multiple of different frequencies, it may be advantageous to record the signal for treatment of the tissues, for example on tape. The recorded signal may then be applied to the electrodes as required. Thus, the signal generator may be replaced by a generator adapted to reproduce a recorded signal, for example a tape recorder may be substituted for the signal generator and may be used to reproduce the recorded signal from a recorded tape. Amplification of the reproduced signal may be required before applying it to the electrodes.

**Claims**

1. Apparatus for influencing biological activity comprising:
   means (3) for generating an oscillating voltage;
   control means (5) for enabling intermittent generation of the oscillating voltage in the form of pulses comprising series of oscillations, the control means being operable so that the duration of the pulses and the duration of the intervals between pulses is independently selectable,
   characterised in that;
   two movable electrodes (1) are connected to the output of the generating means to develop from the oscillating voltage an oscillating electrical field, extending from one electrode to the other, for application to a biological subject either through a liquid medium contacting the biological subject or by direct contact with the biological subject;

the generating means is adapted to produce the oscillating voltage at a frequency of 40 -7,500 Hz;
   and the electrical field oscillations are simple square waves.

2. Apparatus according to claim 1 characterised in that the duration of said pulses and of said intervals between said pulses is in the range of 0.001-10 seconds.

3. Apparatus according to claim 1 or claim 2 characterised in that the output voltage between said electrodes (1) is in the range 2-30 volts.

4. Apparatus according to claim 3 characterised by means for varying said output voltage.

5. Apparatus according to any preceding claim characterised by means for varying the oscillation frequency of said electrical field.

6. Apparatus according to any preceding claim characterised by an amplifier (4) for amplifying the electrical signal to said electrodes.

7. Apparatus according to any preceding claim characterised by means (6) for monitoring the electrical signal supplied to said electrodes (1).

8. Apparatus according to any preceding claim characterised by means for recording the electrical signal applied to said electrodes (1) and means for reproducing said recorded signal.

9. Apparatus according to any preceding claim characterised by a tank (2) for containing said liquid medium.

10. Apparatus according to any preceding claim characterised in that said electrodes (1) comprise an outer layer of material (9) for absorbing said liquid medium.

11. Apparatus according to any preceding claim characterised in that said electrodes (1) are adapted for applying said field through water contained in soil.

12. Apparatus according to any preceding claim characterised in that said liquid medium is water.

**Patentansprüche**

1. Vorrichtung zur Beeinflussung der biologischen Aktivität mit
   - einer Einrichtung (3) zur Erzeugung einer oszillierenden Spannung und
   - einer Regeleinrichtung (5), die eine intermittierende Erzeugung der oszillierenden Spannung in Form von Folgen von Schwingungen umfassenden Impulsen ermöglicht sowie so betätigbar ist, daß die Dauer der Impulse und die Dauer der Intervalle zwischen Impulsen unabhängig wählbar ist,
   dadurch gekennzeichnet, daß
   - zwei bewegbare Elektroden (1) mit dem Ausgang der Erzeugereinrichtung verbunden sind, um aus der oszillierenden Spannung ein sich von der einen Elektrode zur anderen erstreckendes oszillierendes elektrisches Feld für eine Anwendung an einem biologischen Subjekt entweder durch ein das biologische Subjekt berührendes flüssiges Medium oder durch unmittelbaren Kontakt mit dem biologischen Subjekt zu entwickeln,
   - die Erzeugereinrichtung imstande ist, die oszillierende Spannung mit einer Frequenz von 40 - 7500 Hz zu liefern,

- und die Schwingungen des elektrischen Felds einfache Rechteckwellen sind.

2. Vorrichtung nach Anspruch 1, dadurch gekennzeichnet, daß die Dauer der Impulse und der Intervalle zwischen den Impulsen im Bereich von 0,001 - 10 Sekunden liegt.

3. Vorrichtung nach Anspruch 1 oder 2, dadurch gekennzeichnet, daß die Ausgangsspannung zwischen den Elektroden (1) im Bereich von 2 - 30 Volt liegt.

4. Vorrichtung nach Anspruch 3, gekennzeichnet durch eine die Ausgangsspannung verändernde Einrichtung.

5. Vorrichtung nach einem der vorhergehenden Ansprüche, gekennzeichnet durch eine die Schwingungszahl des elektrischen Felds verändernde Einrichtung.

6. Vorrichtung nach einem der vorhergehenden Ansprüche, gekennzeichnet durch einen das elektrische Signal für die Elektroden verstärkenden Verstärker (4).

7. Vorrichtung nach einem der vorhergehenden Ansprüche, gekennzeichnet durch eine das den Elektroden (1) zugeführte elektrische Signal kontrollierende Einrichtung (6).

8. Vorrichtung nach einem der vorhergehenden Ansprüche, gekennzeichnet durch eine das den Elektroden (1) zugeführte elektrische Signal aufzeichnende Einrichtung und durch eine das aufgezeichnete Signal wiedergebende Einrichtung.

9. Vorrichtung nach einem der vorhergehenden Ansprüche, gekennzeichnet durch einen das flüssige Medium enthaltenden Behälter (2).

10. Vorrichtung nach einem der vorhergehenden Ansprüche, dadurch gekennzeichnet, daß die Elektroden (1) eine äußere Schicht (9) eines das flüssige Medium absorbierenden Materials umfassen.

11. Vorrichtung nach einem der vorhergehenden Ansprüche, dadurch gekennzeichnet, daß die Elektroden (1) zu einem Anlegen des Felds durch im Erdboden enthaltenes Wasser imstande sind.

12. Vorrichtung nach einem der vorhergehenden Ansprüche, dadurch gekennzeichnet, daß das flüssige Medium Wasser ist.

**Revendications**

1. Appareil destiné à influencer l'activité biologique comprenant:

des moyens (3) destinés à générer une tension d'oscillation;

des moyens de commande (5) destinés à permettre la génération intermittente de la tension d'oscillation sous la forme d'impulsions comportant des séries d'oscillations, les moyens de commande pouvant être actionnés de manière à ce que la durée des impulsions et la durée des intervalles entre impulsions puissent être sélectionnées indépendamment,

caractérisé en ce que

deux électrodes mobiles (1) sont reliées à la sortie des moyens de génération pour créer à partir de la tension d'oscillation un champ électrique oscillatoire, s'étendant d'une électrode à l'autre, destiné à être appliqué à un sujet biologique soit par l'intermédiaire d'un milieu liquide en contact avec le sujet biologique soit par contact direct avec le sujet biologique;

les moyens de génération sont adaptés à la production de la tension d'oscillation à une fréquence de 40-7500 Hz;

et les oscillations du champ électrique sont des ondes carrées simples.

2. Appareil selon la revendication 1 caractérisé en ce que la durée desdites impulsions et desdits intervalles entre lesdites impulsions se situe dans la gamme allant de 0,001 à 10 secondes.

3. Appareil selon la revendication 1 ou la revendication 2, caractérisé en ce que la tension de sortie entre lesdites électrodes (1) se situe dans la gamme allant de 2 à 30 volts.

4. Appareil selon la revendication 3, caractérisé par des moyens destinés à faire varier ladite tension de sortie.

5. Appareil selon l'une quelconque des revendications précédentes, caractérisé par des moyens destinés à faire varier la fréquence d'oscillation dudit champ électrique.

6. Appareil selon l'une quelconque des revendications précédentes, caractérisé par un amplificateur (4) destiné à amplifier le signal électrique fourni auxdites électrodes.

7. Appareil selon l'une quelconque des revendications précédentes, caractérisé par des moyens (6) destinés à contrôler le signal électrique fourni auxdites électrodes (1).

8. Appareil selon l'une quelconque des revendications précédentes, caractérisé par des moyens destinés à enregistrer le signal électrique appliqué auxdites électrodes (1) et des moyens destinés à reproduire ledit signal enregistré.

9. Appareil selon l'une quelconque des revendications précédentes, caractérisé par un réservoir (2) destiné à contenir ledit milieu liquide.

10. Appareil selon l'une quelconque des revendications précédentes, caractérisé en ce que lesdites électrodes (1) comportent une couche externe de matériau (9) pour absorber ledit milieu liquide.

11. Appareil selon l'une quelconque des revendications précédentes, caractérisé en ce que lesdites électrodes (1) sont adaptées pour l'application dudit champ par l'intermédiaire d'eau contenue dans le sol.

12. Appareil selon l'une quelconque des revendications précédentes, caractérisé en ce que ledit milieu liquide est de l'eau.

F I G.1

_F I G.2_

_F I G.3_